# EUROPEAN PATENT SPECIFICATION

(11) **EP 2 089 352 B1**
(45) Date of publication and mention of the grant of the patent: **10.07.2013**
(21) Application number: 07801739.9
(22) Date of filing: 17.08.2007
(51) Int. Cl.: C07C 67/54, C07C 69/15

(54) **PROCESS FOR WORKING UP VINYL ACETATE**
VERFAHREN ZUR AUFARBEITUNG VON VINYLACETAT
PROCÉDÉ DE PRÉPARATION D'ACÉTATE DE VINYLE

(30) Priority: 17.08.2006 DE 102006038689
(43) Date of publication of application: 19.08.2009
(73) Proprietor: Celanese Chemicals Europe GmbH, 61476 Kronberg (DE)
(72) Inventor: RINNE, Bernd, 65929 Frankfurt (DE); SEHR, Michael, 65551 Limburg (DE); HESS, Stefan, 64521 Gross-Gerau (DE)
(74) Representative: Kador & Partner
(86) International application number: PCT/EP2007/007299
(87) International publication number: WO 2008/019873

(56) References cited:
- EP-A- 0 423 658
- EP-A- 1 760 065
- GB-A- 2 160 520

## Description

The present invention relates to a process for working up vinyl acetate from a gas mixture which is formed in the gas phase in the conversion of ethylene with acetic acid and oxygen over palladium or palladium containing catalysts, in which the energy of the water vapour saturated carbon dioxide discharged from the CO₂-desorber is used.

The production of vinyl acetate by conversion of ethylene with acetic acid and oxygen or oxygen-containing gases over fixed bed catalyst in the gas phase is already known. The reaction is in general carried out at pressures of 1 to 2.5 MPa and temperatures of 100 to 250 °C. Suitable catalysts contain a noble metal portion and an activator portion. The noble metal portion consists of palladium and/or compounds thereof; in addition, gold or compounds thereof can be present. The activator portion consists of compounds of elements of main group 1 and/or main group 2 and/or cadmium. These active components are applied to supports in finely divided form, silica or alumina being in general used as the support material.

In general, the palladium content in the catalyst is between 0.5 and 5 % by weight.

If gold or one of its compounds is used, it is added in an amount of 0.01 to 4 % by weight.

Each individual activator is in general also added in an amount of from 0.01 to 4 % by weight. In all three percentages given, the metal content of the component is in each case based on the overall weight of the supported catalyst. The following catalysts are preferred:

Palladium/alkali metal/cadmium and palladium/gold/alkali metal it being possible for palladium and/or gold to be present in the ready-to-use catalyst as metals or as a compounds, and a preferred alkali-element being potassium. Potassium is used in the form of a carboxylate, in particular as acetate.

Especially preferred are the catalysts palladium acetate/potassium acetate/cadmium acetate and palladium acetate/barium acetoaurate/ potassium acetate.

In the multi-step catalytic process, vinyl acetate and water are formed in equimolar amounts, as shown in the following net equation:

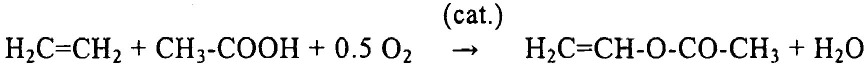

Due to the total oxidation of ethylene, which cannot be avoided completely, CO₂ and water are formed:

H₂C=CH₂ + 3 O₂ → 2 CO₂ + 2 H₂O

Thus, more than 1 mole of water is formed per mole of vinyl acetate; in general the weight of the water formed constitutes about one fourth of the weight of the vinyl acetate formed.

As a further important by-product, CO₂ is formed. The gas mixture leaving the vinyl acetate reactor has a CO₂ content of about 10-20 % by volume. From Ullmann's Encyclopaedia of Industrial Chemistry, Vol. A 27, 5th edition 1996, it is known to absorb the carbon dioxide from the gas stream in a column charged with an aqueous potash solution. The hot potash washing process besides an absorption step also comprises a desorption step, and is known in the field of chemical engineering for the separation of CO₂ from gas streams as Benfield process (Chem.-Ing.-Tech. 60 (1988), No. 5, pages 410-412). In the absorbing section, CO₂ is absorbed in an aqueous potash solution. The pressure in the absorber in general is higher than 1 MPa, so that also a part of the gas to be cleaned dissolves in the washing solution.

The efflux from the CO₂-absorber section can be expanded by use of an intermediate expansion so that the dissolved gas is released in part and can be recycled to the process. The expanded, charged potash solution is fed to the CO₂-desorber section. In the desorber, the CO₂ vapour pressure of the potash solution is increased by heating and CO₂ is expelled by feeding of direct steam or boiling. Usually, the washing solution is regenerated at atmospheric pressure or small overpressure, and the potash solution leaving via the desorber bottom is recycled to the absorber section at a temperature of about 100 to 120 °C. At the head of the CO₂-desorber, CO₂ saturated with water vapour is removed at a temperature of about 100 °C.

In the prior art there is no indication to use the heat content of the water vapour saturated CO₂ obtained at the head of the CO₂-desorber in the working up of vinyl acetate by distillation.

The use of said heat content in the working up of vinyl acetate by distillation is desirable. In the production of vinyl acetate, besides other by-products also ethyl acetate is obtained in an amount of about 500 to 2000 ppm based on the amount of vinyl acetate formed.

Ethyl acetate may be present in pure vinyl acetate in a small amount of 250 ppm only. The separation of ethyl acetate is energy-consuming, and in the art various processes are known which aim to reduce the consumption of energy in the purification of vinyl acetate by separation of ethyl acetate and other by-products. Moreover, also the separation of the remaining acetic acid traces from vinyl acetate is energy-consuming.

In the process according to DE-A1-3 422 575, the hot gas mixture leaving the vinyl acetate reactor, which consists essentially of ethylene, acetic acid, vinyl acetate, water, carbon dioxide, oxygen and inert compounds such as nitrogen and argon, and furthermore contains ethyl acetate, is fed to a first distillation column, the so-called pre-dehydration column, which is operated without additional heating. The gas mixture leaving at the head of this column first is brought into contact with the reflux to the pre-dehydration column in a heat exchanger, so that the gas mixture is cooled and the reflux is heated correspondingly. The gas mixture then is fed from the heat exchanger to a condenser. The portions liquefied therein are trapped in a collecting tank, in which the liquid separates into an aqueous and an organic phase. The aqueous phase is removed and the organic phase completely or in part is recycled as a reflux to the head of the pre-dehydration column.

The portions which are not liquefied in the condenser still contain gaseous vinyl acetate. This vinyl acetate is washed out of the gas mixture in a washing column operated with acetic acid as washing liquid, which is also designated as recycle-gas washer. The remaining gas is recycled to the reactor. The efflux from the bottom of the recycle-gas washer as well as the remained of the organic phase liquefied from the condensate of the pre-dehydration column is collected in a further tank, in case not the complete organic phase liquefied from the condensate is used as reflux for the pre-dehydration column.

At the bottom of the pre-dehydration column a mixture is obtained, which consists of vinyl acetate, acetic acid and about half of the reaction water, as well as by-products. The other half of the reaction water has already been split off without energy supply and forms the aqueous phase of the condensate which is formed upon cooling of the head vapour of the pre-dehydration column.

The bottom product of the pre-dehydration column first is fed to a collecting tank, which is also designated as collecting tank for raw vinyl acetate, and is then worked up in a second distillation column, the so-called azeotrop column. As a head product, water-saturated vinyl acetate is obtained; furthermore an ethyl acetate containing side stream is obtained, and finally a bottom product is obtained, which is recycled to the system as feedback acetic acid. The ethyl acetate containing side stream is removed. The water-saturated vinyl acetate, which is not recycled as reflux to the head of the second distillation column, is combined with the bottom efflux of the recycle gas washer and the remainder of the organic phase liquefied from the condensate of the pre-dehydration column.

Then, the mixture is fed to a third distillation column, the so-called dehydration column. The head vapour of this column after condensation almost completely is recycled as reflux. The removed side stream is separated into an aqueous and an organic phase, and the aqueous phase is removed and the organic phase is recycled to the column. From the bottom of the dehydration column, a dry vinyl acetate/acetic acid mixture is removed and is fed to a further, fourth column, the so-called pure vinyl acetate distillation column. In this column, as a head product vinyl acetate which is almost completely free of ethyl acetate is obtained, and the bottom product of this column, which contains acetic acid, high boiling compounds and traces of vinyl acetate and ethyl acetate, is recycled to the process with a partial stream being removed.

A further variant for working up of vinyl acetate is known from EP-A2-0 423 658. In this process, the bottom product of the recycle gas washer is not directly combined with water-containing vinyl acetate obtained from the azeotrop column, but is first fed to a further column, in which as a head product a vinyl acetate-water azeotrop is obtained, and as a bottom product acetic acid is obtained which is recycled to the process. The aqueous vinyl acetate obtained in this additional column is combined with the water-saturated vinyl acetate obtained from the azeotrop column and is further worked up in the subsequent dehydration column and pure vinyl acetate distillation column, in accordance with the process of DE-A1-3 422 575. The process according to EP-A2-0 423 658 requires about the same consumption of distillation energy for the separation of ethyl acetate as the process according to DE-A1-3 422 575, however, a smaller number of column plates is required and thus the investment costs are lower. The noncondensed part of the vinyl acetate from the pre-dehydration column which is washed out in the recycle gas washer with acetic acid and is obtained as acetic solution as well as the organic phase of the condensate from the pre-dehydration column are almost free of ethyl acetate, and thus an energetically expensive separation of ethyl acetate from said vinyl acetate streams is not necessary. However, this process variant requires the operation of an additional distillation column for the splitting off of the bottom efflux from the recycle gas washer.

It is characteristic for the vinyl acetate working up processes of DE-A1-3 422 575 and EP-A2-0 423 658 that the acetic solution obtained from the bottom efflux of the recycle gas washer, the water-saturated vinyl acetate from the head product of the azeotrop column, and the remainder of the organic phase liquefied from the condensate of the pre-dehydration column are combined. Thus, to the further purification stages performed in the subsequent dehydration column and pure vinyl acetate distillation column, an acetic mixture is fed from which acetic acid has to be separated, thus requiring a high consumption of energy. Furthermore, the dehydration column and the pure vinyl acetate distillation column must be designed with corrosion-resistant material insensitive to acetic acid.

Still further, the condensate of the pre-dehydration column, which is not recycled as reflux to the head of the pre-dehydration column, still contains a certain amount of ethyl acetate. Because this stream is combined with the water-saturated vinyl acetate only after the azeotrop column, which is obtained as a head product from said column, an ethyl acetate containing stream is fed to the subsequent dehydration column and pure vinyl acetate distillation column, from which ethyl acetate can be separated only in an energy-consuming process.

In the non-published German patent application with the file number 10 2005 036 930.8-44, in contrast to the processes known from DE 34 22 575 A1 and EP 0 423 658 A2 the bottom efflux from the recycle gas washer is not fed to a collecting tank to which further streams are fed and which are then together fed to the dehydration column and pure vinyl acetate distillation column, but the bottom efflux from the recycle gas washer after heating is introduced into the lower part of the pre-dehydration column, thus further heating the mentioned acetic bottom efflux from the recycle gas washer. This measure inter alia leads to a washing effect in the pre-dehydration column, so that almost the complete ethyl acetate is washed into the bottom of the pre-dehydration column and is removed. Furthermore, by this measure no acetic acid containing stream is fed to the collecting tank before the dehydration column, but the acetic bottom efflux from the pre-dehydration column is directly introduced into the azeotrop column. The complete acetic acid is removed with the bottom of the azeotrop column, so that the stream supplied to the dehydration column and thus also the stream supplied to the pure vinyl acetate distillation column is free of acetic acid. With the stream supplied to the pure vinyl acetate distillation column being free of acetic acid, also the distillation expenditure for obtaining the pure vinyl acetate is decreased, because the separation of traces of acetic acid from vinyl acetate is very costly.

In the vinyl acetate distillation process described in the non-published German patent application with the file number 10 2005 036 930.8 distillation is carried out at a lower temperature. The bottom temperature in the pure vinyl acetate distillation column in general is about 80 °C, in contrast to the temperature of 120-135 °C in the process according to DE 34 22 575 A1 and EP 0 423 658 A2.

The process for working up of vinyl acetate according to the teaching of the non-published German patent application with the file number 10 2005 036 930.8 can still be improved, if the CO₂ saturated with water vapour, which is obtained at the head of the CO₂-desorber and has a temperature of about 100 °C, is used for the heating of the pure vinyl acetate distillation column. The decisive measure for the use of the heat content of the CO₂ saturated with water vapour, which is obtained at the head of the CO₂-desorber and has a temperature of about 100 °C, for the heating of the pure vinyl acetate distillation column is the lowering of the bottom temperature of the pure vinyl acetate distillation column. Such a lowering of the temperature is possible without a decrease in the quality of the pure vinyl acetate because the complete acetic acid is removed via the bottom of the azeotrop column, and thus a supply of an acetic acid containing stream to the dehydration column and the pure vinyl acetate distillation column is avoided. Through the lowering of the temperature in the bottom of the pure vinyl acetate distillation column, a sufficient difference to the temperature of the water-saturated CO₂ which is obtained at the head of the CO₂-desorber is built up, which makes it possible to use the heat of the water vapour-saturated CO₂, which can also be designated as desorber exhaust vapour, for the heating of the pure vinyl acetate distillation column.

The invention thus consists in a process for the separation of vinyl acetate from the gas mixture being formed in the gas phase in the conversion of ethylene with acetic acid and oxygen over palladium or palladium containing catalysts, wherein
a) the gas mixture leaving the reaction zone is introduced into a first distillation column,
b) the gas mixture leaving at the head of the first distillation column is cooled to -20 to +50 °C, so that a condensate is formed which separates into an aqueous phase and an organic phase,
c) the aqueous phase formed in step b) is removed,
d) the organic phase formed in step b) completely or in part is reintroduced as reflux to the head of the first distillation column used in step a) and a part of the organic phase not used as reflux is removed,
e) the gas not condensed in step b) which contains vinyl acetate is washed in a washing column with at least 90% aqueous acetic acid to yield a vinyl acetate containing acetic solution at the bottom,
f) a portion of the bottom product of the washing column used in step e) is first cooled during pumping over and is then reintroduced into the bottom of the washing column used in step e), the remaining part of the bottom product is removed and heated to at least 30 °C, and then fed to the lower part of the first distillation column used in step a),
g) a gas stream is removed at the head of the washing column of step e), from which a portion is discharged as exhaust and the remaining portion is recycled to the reaction zone,
h) the exhaust discharged in step g) is passed through an aqueous, alkaline solution,
i) the aqueous, alkaline solution obtained in step h) is regenerated by increasing the temperature to 100-120°C,
j) the CO₂ stream saturated with water vapour resulting from step i) is removed,
k) the vinyl acetate, ethyl acetate, acetic acid and water containing bottom product of step a) is fed to a collecting tank and the pressurized liquid is expanded so that a gas is formed,
l) the liquid which results from the expansion in step k) is fed to a second distillation column, and from a concentration zone above its bottom an ethyl acetate containing side stream is removed,
m) the acetic acid and water containing bottom product of step 1) is used completely or in part for the washing of the gas in step e),
n) the head vapour of step 1) is cooled, so that a condensate is formed which separates into an aqueous and an organic phase,
o) the aqueous phase formed in step n) is removed,
p) a portion of the organic phase formed in step n) is recycled as reflux to the head of the second distillation column used in step 1), and the remaining portion is removed,
q) the remaining organic phase removed in step d) is expanded, and the gas formed during expansion is combined with the gas formed in step k), and the combined gas streams are recycled to the process,
r) the organic phase resulting from step q) is combined with the organic phase resulting from step n), and the remaining removed portion of the organic phase of step p), which is not used as reflux, is introduced into a third distillation column,
s) the head product of the third distillation column of step r) is cooled and the resulting low boiling fraction and the resulting water are separated,
t) the bottom product of the third distillation column of step r) is introduced into a fourth distillation column,
u) pure vinyl acetate is removed from the head of the fourth distillation column used in step t), characterised in that
v) the bottom product of the fourth distillation column is removed, and a portion thereof is heated in one or more heat exchangers, which are operated with the water vapour-saturated CO₂ stream obtained in step j), and
w) the so heated bottom product is recycled to the lower part of the fourth distillation column.

In step a), preferably the gas mixture leaving the reaction zone first is cooled 115 °C to 150 °C in counter current heat exchange with the colder recycle gas, which is thus heated and then recycled to the reaction. In this step, a condensation of the liquefiable portions does not yet occur and the gas mixture is introduced into the first distillation column, also designated as pre-dehydration column.

The amount of the organic phase formed in step b) depends on the temperature to which the gas mixture is cooled down in this step. The portion of the organic phase of step b) which is not used as reflux in step d) is removed and is expanded in step q) from a first pressure stage at 0.5 to 2.0 MPa to a second pressure stage at 0.1 to 0.3 MPa, preferably 0.1 to 0.15 MPa. The resulting liquid is combined in step r) with the organic phase from the condensed head product of the second distillation column, also designated as azeotrop column (step n). Preferably, both organic phases are combined in the phase separator of the azeotrop column. The portion of the organic phase which is not introduced as reflux to the head of the azeotrop column is introduced into a third column, which is also designated as dehydration column.

Preferably, the cooling temperature in step b) and the portion of the organic phase formed in step b) and used as a reflux in step d) is selected so that the bottom product of step a) contains an amount of vinyl acetate which is as small as possible and contains, as far as possible, the complete amount of ethyl acetate.

After step e), a portion of the bottom efflux of the washing column, which is also designated as recycle gas washer, is pumped over, and the portion of the bottom product of the washing column which is pumped over is cooled. For cooling of the bottom product, means are used which are known to the skilled person, for example heat exchangers. The portion of the bottom efflux which is not pumped over is removed from the washing column and is heated to a temperature of at least 30 °C, preferably of 60 to 120 °C, in particular of 60 to 100 °C, and is then introduced to the lower part of the first distillation column used in step a). Suitably, for heating the bottom product pumped out of the washing column it is passed through a heat exchanger.

Preferably, the mentioned bottom efflux of step f) is fed to the 2. to 15., in particular 5. to 10., column plate of the first distillation column, counted from the bottom of the column.

At the head of the washing column, a gas stream is removed, which contains ethylene, non-reacted oxygen and, as a by-product, carbon dioxide. From this gas stream, a portion is removed as exhaust, and the rest is recycled to the reaction zone.

The removed exhaust contains carbon dioxide and is submitted to a washing step with an aqueous, alkaline solution for removal of the carbon dioxide.

Such kind of exhaust washing steps are known in the art. Carbon dioxide first is absorbed in an aqueous potash solution and is thus removed from the exhaust. In a subsequent desorption step, the potash solution is regenerated by raising the temperature to 100-120 °C. The hot aqueous potash solution is passed back to the absorber section, and at the head of the desorber water-saturated CO₂ is removed at a temperature of, preferably, 90-120°C and at a pressure of, preferably, 0.1 to 0.12 MPa (step j). The removed, heated mass stream is also designated as desorber exhaust vapour.

The head product of the first distillation column contains only very small amounts of ethyl acetate, and the recycled reflux of step d) and the portion of the organic phase which is not used as reflux are poor in ethyl acetate and thus can be further processed without further measures which would require a separation of ethyl acetate. For further processing, the removed organic phase is expanded according to step q) and the resulting liquid is combined with the organic phase resulting from step n), which is obtained from the head product of the second distillation column, also designated as azeotrop column. The combined organic phases in part are recycled as reflux to the head of the azeotrop column. The remainder is fed to a third distillation column, which is also designated as dehydration column (step r).

The gas resulting from the expansion in step q) is also designated as feedback gas and has about the same composition as the feedback gas resulting from the expansion of the bottom efflux of the first distillation column in step k). Both feedback gas streams are combined, compressed in a feedback gas compressor and then recycled to the process. Suitably, the combined feedback gas is further combined with the residual gas resulting from the acetic acid washing in step e), which is also designated as recycle gas. The combined gas streams are compressed and are recycled to the vinyl acetate reactor after discharge of a portion containing inert compounds.

In the gas washing of step e), at least in part the bottom product of the second distillation column is used (step 1). The bottom product mainly consists of acetic acid and further contains water in an amount of at most 10 % by weight. A portion of the bottom product not needed in step e) preferably is recycled to the reactor as feedback acetic acid, after a small portion thereof has been discharged for removal of high boiling compounds and polymers.

In step r), preferably only such an amount of the combined organic phases of step n) and q) is recycled as reflux, so that the head vapour of the second distillation column contains ethyl acetate in an amount as small as possible. The portion of the organic phase, which is not needed for this purpose, is introduced into the third distillation column, which is also designated as dehydration column.

In step s), the condensed head product of the third distillation column is not completely used as reflux, but a portion sufficient for the separation of low boiling compounds and water is removed.

In step t), the bottom efflux of the third distillation column, which virtually consists of dry vinyl acetate, is fed to a fourth distillation column, the so-called pure vinyl acetate distillation column, from which pure vinyl acetate is removed as a head product (step u).

Through recycling of the bottom product from the washing column, which is not pumped over, to the lower part of the first distillation column used in step a (step e) an acetic acid free stream is fed to the pure vinyl acetate distillation column. Thus, the pure vinyl acetate distillation column can be operated at a temperature of 70 to 100 °C, preferably 70 to 90 °C, without a decrease in the quality of the vinyl acetate. Said lowering of the temperature causes a sufficient temperature difference between the pure vinyl acetate distillation column to be operated and the water vapour-saturated carbon dioxide, also designated as desorber exhaust vapor, removed according to step j). This temperature difference is sufficient to use the heat content of the desorber exhaust vapor for heating of the pure vinyl acetate distillation column.

In accordance with the invention, a bottom efflux is removed from the pure vinyl acetate distillation column at a temperature of 70 to 100 °C, preferably 70 to 90 °C. A portion of this bottom efflux is passed through one or several heat exchangers and is thus heated to a temperature of 80 to 110 °C, preferably 80 to 100 °C. The so heated bottom product is recycled to the lower part of the pure vinyl acetate distillation column where it is evaporated. For the operation of the heat exchangers used for heating of said pump over stream, the desorber exhaust vapor with a temperature of 90 to 120 °C is used. The amount pumped over is selected so that the energy of said desorber exhaust vapor is optimally used. The portion of the bottom efflux of the pure vinyl acetate distillation column which is not pumped over is fed to the low part of the azeotrop column operated in step a). The process according to invention makes is possible to use the heat content of the desorber exhaust vapor for heating of the pure vinyl acetate distillation column thus leads to a significant saving of energy in this vinyl acetate working up process.

The first, second, third and fourth distillation column in the claimed vinyl acetate working up process are operated at such temperature, pressure and feed back conditions which result from the overall performance of the plant.

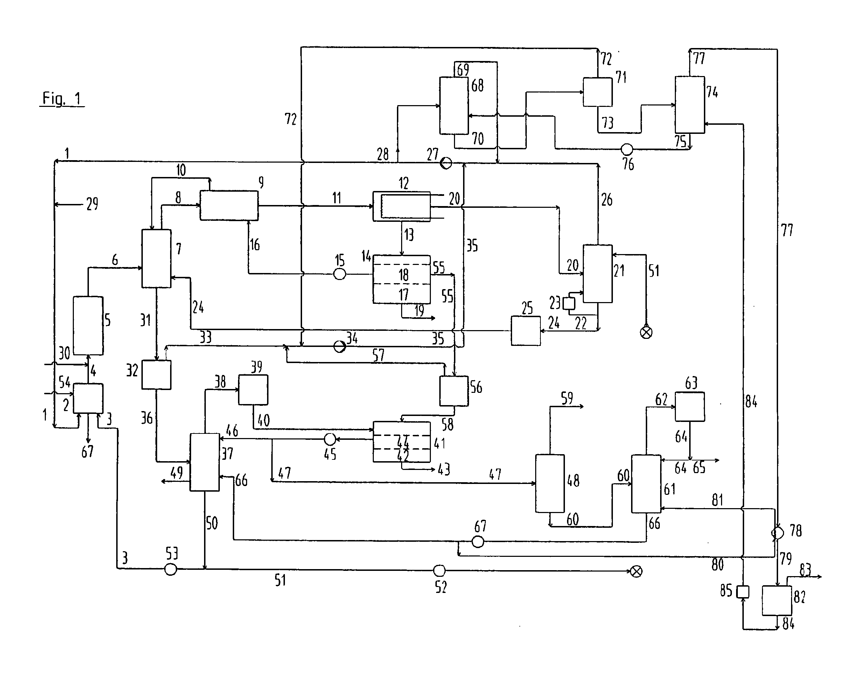

The process according to the invention is illustrated in Figure 1. Known measures such as addition of stabilizer are not shown.

The recycled gas mixture, also designated as recycle gas, consisting of ethylene, oxygen and CO₂, as well as inert compounds and low amounts of organic compounds such as acetic acid is passed via line (1) into an acetic acid evaporator (2) designed as plate column in which the gas stream is charged with acetic acid fed in via line (3). The gas mixture leaving the acetic acid evaporator (2) is fed into the vinyl acetate reactor (5) via a vapour heated line (4).

The gas mixture leaving the vinyl acetate reactor (5), which is mainly consisting of ethylene, acetic acid, vinyl acetate, water, carbon dioxide, oxygen as well as inert gases such as nitrogen and argon, is passed via line (6) into the first distillation column, the pre-dehydration column (7). The pre-dehydration column (7) is designed in a known manner.

The gas mixture leaving the pre-dehydration column (7) at the head enters a heat exchanger (9) via line (8) where it is brought into counter current heat exchange with the reflux which enters via line (16) and is recycled to the pre-dehydration column (7) via line (10). From the heat exchanger (9) the gas mixture enters a water-cooled condenser (12) via line (11), where it is cooled to about 35 °C. The liquefied portions enter tank (14) via line (13), where they are collected. The portion of liquid exceeding a certain level in the collecting tank (14) is pumped back into the pre-dehydration column (7) by means of pump (15) via line (16), heat exchanger (9) and line (10). After a certain period of time, the condensate formed in the collecting tank (14) separates into two phases (17) and (18); from now on, the aqueous phase (17) is discharged via line (19) and only the organic phase (18) is pumped back as reflux into the head of the pre-dehydration column (7) completely or in part via line (16), heat exchanger (9) and line (10).

The gas mixture leaving the condenser (12) via line (20) is washed with the acetic acid coming from line (51) in washing column (21) (recycle gas washer) and is freed from uncondensed vinyl acetate portions. The bottom efflux from the recycle gas washer (21) is divided, with a partial stream being pumped over under cooling by means of heat exchanger (23) via line (22) and being recycled to the recycle gas washer, and the other portion of the bottom efflux being passed through a heat exchanger (25) via line (24) in which the bottom efflux is heated to a temperature of at least 30 °C, preferably 60 to 120 °C, and in particular 60 to 100 °C. The thus heated bottom efflux then is pumped back to the lower part of the pre-dehydration column, preferably to the 2. to 15., in particular to the 5. to 10. plate, calculated from the column bottom.

The residual gas or recycle gas (ethylene, unconverted oxygen and CO₂ formed as by-product) leaving the washing column (21) via line (26) is combined with the feedback gas fed via line (35), which predominately contains ethylene and, moreover, CO₂, inert compounds such as nitrogen and argon, as well as acetic acid and small amounts of vinyl acetate and ethyl acetate, and the combined gas streams are compressed by means of recycle gas compressor (27) and are recycled to reactor (5) via line (1) and acetic acid evaporator (2). A portion of the recycle gas is removed as a waste gas via line (28) in order to discharge inert components. Fresh ethylene is fed via line (29) and fresh oxygen is fed via line (30).

The liquid obtained at the bottom of the pre-dehydration column (7), consisting mainly of vinyl acetate, acetic acid and water and containing almost the complete ethyl acetate, is fed to a tank (32), which is also designated as row vinyl acetate collecting tank, via line (31), and is expended, preferably to a pressure of 0.1 to 0.3 MPa, in particular to a pressure of 0.1 to 0.15 MPa. The feedback gas formed during expansion, which contains predominantly ethylene and, moreover, CO₂, inert compounds such as nitrogen and argon, as well as organic components such as acetic acid, is discharged via line (33), then is combined with the feedback gas fed via line (57) which has about the same composition, and finally, after having been compressed in feedback gas compressor (34), is combined via line (35) with the recycle gas from the recycle gas washer (21) fed via line (26). The organic phase obtained after expansion in the raw vinyl acetate collecting tank (32) is discharged via line (36) and fed into the second distillation column (37), which is also designated as azeotrop column.

The head vapor of the second distillation column (37) is fed to cooler (39) via line (38) and condensed therein. The condensate which is passed to phase separator (41) via line (40) separates into an aqueous phase (42) which is removed via line (43), and an organic phase (44) which is combined with the organic phase fed via line (58). The combined organic phase in phase separator (41) is discharged by means of pump (45). A portion of the discharged organic phase is fed to the head of the azeotrop column (37) via line (46) and is used there as reflux. The portion not used as reflux is discharged via line (47) and fed to a third distillation column (48), the dehydration column. The ethyl acetate entering via line (36) into column (37) is discharged from a concentration zone above the bottom of column (37) via line (49). The bottom product of column (37) contains almost the complete acetic acid obtained in the vinyl acetate working up stage, at most 10 % water, small amounts of high boiling components and/or polymers, and only traces of vinyl acetate and ethyl acetate.

The aqueous acetic acid is discharged from the bottom of column (37) via line (50) and divided. Depending on the design of the washing column (21) and the temperature of the gases to be washed, differing amounts of acetic acid are necessary as washing liquid. The portion necessary for the acetic acid washing in step e) is fed to washing column (21) via line (51) and pump (52). The remainder is fed to the acetic acid evaporator (2) via pump (53) and line (3). Fresh acetic acid is fed via line (54) to the head of acetic acid evaporator (2) in an amount corresponding to the amount of acetic acid consumed during the reaction, and simultaneously serves as washing solution for the recycled acetic acid fed via line (3), which is also designated as feedback acetic acid.

The remainder of the organic phase (18) from collecting tank (14) is passed to expansion tank (56) via line (55), in case not the entire organic phase (18) is used as reflux in the pre-dehydration column (7). The feedback gas formed during expansion to a pressure of 0.02 to 0.2 MPa, preferably to 0.1 to 0.15 MPa, is discharged via line (57), is combined with the feedback gas fed via line (33), and is recycled back to the process via line (35) after compression by means of the feedback gas compressor (34).

The liquid obtained in tank (56) is fed to phase separator (41) via line (58) wherefrom the combined organic phases in part are fed to the azeotrop column (37) via line (46) as reflux, and in part as are fed via line (47) as supply to the third distillation column (48), which is also designated as dehydration column. The supply to the dehydration column is virtually free of acetic acid.

The low boiling compounds and the remaining water residues contained in the head vapor of column (48) are removed via line (59) and are discharged from the working up process.

The virtually water-free vinyl acetate which is obtained at the bottom of column (48) is passed via line (60) to the fourth distillation column (61), which is also designated as pure vinyl acetate distillation column. The head vapor of this column is fed to condenser (63) via line (62). The obtained condensate is pure vinyl acetate free of ethyl acetate. A very small portion of said vinyl acetate is fed as reflux to column (61) via line (64). Pure vinyl acetate is discharged via line (65). The bottom product of column (61) which contains small amounts of ethyl acetate, polymers and high boiling compounds is in part recycled to column (37) via line (66) and pump (67).

A partial stream is removed via line (67) from acetic acid evaporator (2), into which finally all pipe volume components and polymers are recycled, to discharge the polymers.

The exhaust removed via line (28) is passed to an absorption column (68) charged with a 20 to 40 % by weight aqueous, alkali carbonate solution, preferably potassium carbonate solution, and from the head of the adsorption column (68) exhaust is removed via line (69) which is free from carbon dioxide. The purified exhaust is recycled to the process and is fed to line (26). The bottom of the absorption column removed via line (70) is expanded in tank (71) from a pressure stage of 0.6 to 1.3 MPa to a pressure stage of 0.12 to 0.3 MPa, and gaseous portions are recycled to the process via line (72) and are fed to line (33). The liquid portions are fed to the desorption column (74) via line (73), to which energy is supplied by means of heat exchangers. The hot regenerated alkali carbonate solution is passed back to the absorption column (68) via the bottom of the desorption column (64) and line (65) by means of pump (76). The desorption and absorption columns are designed in a known manner. The carbon dioxide which is saturated with water vapor and has a temperature of 90 to 120 °C is removed at a pressure of 0.1 to 0.12 MPa via line (77) and is used for the operation of the heat exchanger (78). The water vapor-saturated carbon dioxide cooled in the heat exchangers (78) then is removed via line (79). In general, one or more heat exchangers (78) are operated by use of the desorber exhaust vapours fed via line (77). The heat exchangers operated in the process according to the invention are designed in known manner.

The desorber exhaust vapours fed via line (77) have a temperature of 90 to 120 °C and are cooled in heat exchangers (78) to about 80 to 90 °C.

From the bottom product removed via line (66) a portion is derived after pump (67) and is fed to the heat exchangers (78) operated with the desorber exhaust vapor via line (80).

The bottom product removed from column (61) has a temperature of 70 to 100 °C, preferably 70 to 90 °C, and is heated in heat exchangers (78) to a temperature of 80 to 110 °C, preferably 80 to 100 °C. The heated pumped over stream then is recycled via line (81) to the lower part, preferably to at most the third column plate calculated from the column bottom, and, in particular, to the bottom of the pure vinyl acetate distillation column, and is evaporated there.

The measure essential for the working up process according to the invention consists in the heating of a portion of the bottom product from the pure vinyl acetate distillation column (61) which is pumped over, with the desorber exhaust vapours in heat exchangers (78) fed via line (77). By this measure, the bottom product is heated from a temperature of preferably 70 to 90 °C to a temperature of 80 to 110 °C, preferably 80 to 100 °C. The thus heated bottom product is recycled to the pure vinyl acetate distillation column and evaporated there.

The water vapor-saturated CO₂ stream cooled in heat exchangers (78) and removed therefrom via line (79) is further cooled in a water cooled condenser (82). The CO₂ gas stream resulting therefrom is removed via line (83) and finally discharged. The liquid phase obtained in condenser (82) is removed via line (84) and recycled to the desorption column (74).

The measure according to the invention, namely the heating of the bottom efflux from the pure vinyl acetate distillation column by means of the desorber exhaust vapour furthermore makes possible an elegant analytical control of the operation of the pure vinyl acetate distillation column. For this purpose, in line (84) an additional container (85) can be installed as a control container. In case during the operation of the pure vinyl acetate distillation column there is a breaking through of vinyl acetate, in container (85) two liquid phases are formed and the occurrence of a phase separation can be realized in a simple manner during the operation of the plant.

Furthermore, by means of the control of the pressure conditions in the pure vinyl acetate distillation column (61) and in the desorption column (74), i.e. by means of a pressure increase in the desorption column (74) and/or by means of a pressure decrease in the pure vinyl acetate distillation column (61) the temperature difference between the bottom efflux of the pure vinyl acetate distillation column and the desorber exhaust vapours can be controlled. Thus, the optimum use of the energy content of the desorber exhaust vapours can be achieved in a simple manner by means of a purposeful control of the pressure conditions in the pure vinyl acetate distillation column (61) and/or in the desorption column (74).

By means of the use of the heat content of the desorber exhaust vapours for the heating process in the pure vinyl acetate distillation column the heat consumption for its operation can significantly be decreased without that there is a lowering in quality, so that the mode of operation according to the invention leads to a significant saving of energy during the operation of the pure vinyl acetate distillation column.

## Claims

1. A process for the separation of vinyl acetate from a gas mixture formed in the gas phase in the conversion of ethylene with acetic acid and oxygen over palladium or palladium containing catalysts, wherein
a) the gas mixture leaving the reaction zone is introduced into a first distillation column,
b) the gas mixture leaving at the head of the first distillation column is cooled to -20 to +50 °C, so that a condensate is formed which separates into an aqueous phase and an organic phase,
c) the aqueous phase formed in step b) is removed,
d) the organic phase formed in step b) completely or in part is reintroduced as reflux to the head of the first distillation column used in step a) and a part of the organic phase not used as reflux is removed,
e) the gas not condensed in step b) which contains vinyl acetate is washed in a washing column with at least 90% aqueous acetic acid to yield a vinyl acetate containing acetic solution at the bottom,
f) a portion of the bottom product of the washing column used in step e) is first cooled during pumping over and is then reintroduced into the bottom of the washing column used in step e), the remaining part of the bottom product is removed and heated to at least 30 °C, and then fed to the lower part of the first distillation column used in step a),
g) a gas stream is removed at the head of the washing column of step e), from which a portion is discharged as exhaust and the remaining portion is recycled to the reaction zone,
h) the exhaust discharged in step g) is passed through an aqueous, alkaline solution,
i) the aqueous, alkaline solution obtained in step h) is regenerated by increasing the temperature to 100-120°C,
j) the CO₂ stream saturated with water vapour resulting from step i) is removed,
k) the vinyl acetate, ethyl acetate, acetic acid and water containing bottom product of step a) is fed to a collecting tank and the pressurized liquid is expanded so that a gas is formed,
l) the liquid which results from the expansion in step k) is fed to a second distillation column, and from a concentration zone above its bottom an ethyl acetate containing side stream is removed,
m) the acetic acid and water containing bottom product of step l) is used completely or in part for the washing of the gas in step e),
n) the head vapour of step l) is cooled, so that a condensate is formed which separates into an aqueous and an organic phase,
o) the aqueous phase formed in step n) is removed,
p) a portion of the organic phase formed in step n) is recycled as reflux to the head of the second distillation column used in step 1), and the remaining portion is removed,
q) the remaining organic phase removed in step d) is expanded, and the gas formed during expansion is combined with the gas formed in step k), and the combined gas streams are recycled to the process,
r) the organic phase resulting from step q) is combined with the organic phase resulting from step n), and the remaining removed portion of the organic phase of step p), which is not used as reflux, is introduced into a third distillation column,
s) the head product of the third distillation column of step r) is cooled and the resulting low boiling fraction and the resulting water are separated,
t) the bottom product of the third distillation column of step r) is introduced into a fourth distillation column,
u) pure vinyl acetate is removed from the head of the fourth distillation column used in step t), **characterised in that**
v) the bottom product of the fourth distillation column is removed, and a portion thereof is heated in one or more heat exchangers, which are operated with the water vapour- saturated CO₂ stream obtained in step j), and
w) the so heated bottom product is recycled to the lower part of the fourth distillation column.

2. Process according to claim 1 **characterized in that** the CO₂ stream saturated with water vapour used in step v) has a temperature of 90-120 °C.

3. Process according to claim 1 or 2 **characterized in that** the CO₂ stream saturated with water vapour used in step v) has a pressure of 0.1 to 0.12 MPa.

4. Process according to one or more of claims 1 to 3 **characterized in that** the fourth distillation column is operated at a temperature from 70 to 100 °C.

5. Process according to one or more of claims 1 to 4 **characterized in that** the bottom product of the fourth distillation column resulting from step v) is removed at a temperature of 70 to 100 °C.

6. Process according to claim 5 **characterized in that** a portion of the resulting bottom product of the fourth distillation column is heated to a temperature of 80 to 110°C.

## Patentansprüche

1. Ein Verfahren zur Abtrennung von Vinylacetat, das in der Gasphase während der Umsetzung von Ethylen mit Essigsäure und Sauerstoff über Palladium oder einem Palladium-enthaltenden Katalysator gebildet wird, aus einer Gasmischung, wobei
a) die Gasmischung, welche die Reaktionszone verlässt, in eine erste Destillationskolonne eingeleitet wird,
b) die Gasmischung, welche die erste Destillationskolonne am Kopf verlässt, auf - 20 bis +50°C gekühlt wird, sodass ein Kondensat gebildet wird, welches sich in eine wässrige Phase und eine organische Phase auftrennt,
c) die wässrige Phase, gebildet in Schritt b), entfernt wird,
d) die organische Phase, gebildet in Schritt b), vollständig oder teilweise wieder als Rücklauf zum Kopf der ersten Destillationskolonne, verwendet in Schritt a), zugeführt wird und ein Teil der organischen Phase, welcher nicht als Rücklauf verwendet wird, entfernt wird,
e) das nicht kondensierte Gas aus Schritt b), welches Vinylacetat enthält, in einer Waschkolonne mit mindestens 90% wässriger Essigsäure gewaschen wird, um eine Vinylacetat-enthaltende Säurelösung im Sumpf zu erhalten,
f) ein Teil des Sumpfprodukts der Waschkolonne, verwendet in Schritt e), zuerst während des Umpumpens gekühlt wird und dann wieder in den Sumpf der Waschkolonne, verwendet in Schritt e), zugeführt wird, der übrig gebliebene Teil des Sumpfprodukts entfernt und auf mindestens 30°C erhitzt und dann in den unteren Teil der ersten Destillationskolonne, verwendet in Schritt a), zugeführt wird,
g) ein Gasstrom am Kopf der Waschkolonne aus Schritt e) entfernt wird, von welchem ein Teil als Abgas abgeführt wird und der restliche Teil in die Reaktionszone rückgeführt wird,
h) das abgeführte Abgas aus Schritt g) durch eine wässrige, alkalische Lösung geleitet wird,
i) die wässrige, alkalische Lösung, erhalten in Schritt h), durch Erhöhung der Temperatur auf 100-120°C regeneriert wird,
j) der mit wasserdampfgesättigte CO₂-Strom aus Schritt i) entfernt wird,
k) das Vinylacetat-, Ethylacetat-, Essigsäure- und Wasser-beinhaltende Sumpfprodukt aus Schritt a) in einen Sammeltank zugeführt wird und die unter Druck stehende Flüssigkeit expandiert wird, sodass ein Gas entsteht,
l) die Flüssigkeit, welche aus der Expansion in Schritt k) resultiert, in eine zweite Destillationskolonne zugeführt wird und aus einer Konzentrationszone oberhalb des Sumpfes ein Ethylacetat-enthaltender Nebenstrom entfernt wird,
m) das Essigsäure und Wasser beinhaltende Sumpfprodukt aus Schritt 1) vollständig oder teilweise zum Waschen des Gases in Schritt e) verwendet wird,
n) der Kopfdampf aus Schritt 1) gekühlt wird, so dass sich ein Kondensat bildet, welches sich in eine wässrige und eine organische Phase auftrennt,
o) die wässrige Phase, die sich in Schritt n) bildet, entfernt wird,
p) ein Teil der organischen Phase, gebildet in Schritt n), als Rücklauf in den Kopf der zweiten Destillationskolonne, verwendet in Schritt l), rückgeführt wird und der restliche Teil entfernt wird,
q) die restliche organische Phase, entfernt in Schritt d), expandiert wird und das während der Expansion gebildete Gas mit dem Gas, gebildet in Schritt k), vereinigt wird und die vereinigten Gasströme in den Prozess rückgeführt werden,
r) die organische Phase, die aus Schritt q) resultiert, mit der organischen Phase, die aus Schritt n) resultiert, und dem restlichen, entfernten Teil der organischen Phase aus Schritt p), welche nicht als Rückfluss verwendet wird, vereint wird und in eine dritte Destillationskolonne eingeführt wird,
s) das Kopfprodukt der dritten Destillationskolonne aus Schritt r) gekühlt wird und die resultierende niedrig siedende Fraktion und das resultierende Wasser aufgetrennt werden,
t) das Sumpfprodukt der dritten Destillationskolonne aus Schritt r) in eine vierte Destillationskolonne eingeführt wird,
u) reines Vinylacetat vom Kopf der vierten Destillationskolonne, verwendet in Schritt t), entfernt wird, charakterisiert dadurch,
v) dass das Sumpfprodukt der vierten Destillationskolonne entfernt wird und der Rest hiervon in einem oder mehreren Wärmetauschern, welche mit dem wasserdampfgesättigten CO₂-Strom aus Schritt j) betrieben werden, erhitzt wird und
w) dass so erhitzte Sumpfprodukt in den unteren Teil der vierten Destillationskolonne rückgeführt wird.

2. Verfahren gemäß Anspruch 1 dadurch charakterisiert, dass der mit wasserdampfgesättigte CO₂-Strom, verwendet in Schritt v), eine Temperatur von 90-120°C hat.

3. Verfahren gemäß Anspruch 1 oder 2 dadurch charakterisiert, dass der wasserdampfgesättigte CO₂-Strom, verwendet in Schritt v), einen Druck von 0.1 bis 0.12 MPa hat.

4. Verfahren gemäß eines oder mehrerer der Ansprüche 1 bis 3 dadurch charakterisiert, dass die vierte Destillationskolonne bei einer Temperatur von 70 bis 100°C betrieben wird.

5. Verfahren gemäß eines oder mehrerer der Ansprüche 1 bis 4 dadurch charakterisiert, dass das Sumpfprodukt der vierten Destillationskolonne, das aus Schritt v) resultiert, bei einer Temperatur von 70 bis 100°C entfernt wird.

6. Verfahren gemäß Anspruch 5 dadurch charakterisiert, dass ein Teil des Sumpfprodukts der vierten Destillationskolonne auf eine Temperatur von 80 bis 110°C erhitzt wird.

## Revendications

1. Procédé de séparation d'acétate de vinyle d'un mélange gazeux formé dans la phase gazeuse lors de la conversion d'éthylène avec de l'acide acétique et de l'oxygène sur du palladium ou des catalyseurs contenant du palladium, dans lequel
a) le mélange gazeux quittant la zone de réaction est introduit dans une première colonne de distillation,
b) le mélange gazeux sortant de la tête de la première colonne de distillation est refroidi à -20 à +50 °C, de façon à former un condensat qui se divise en une phase aqueuse et une phase organique,
c) la phase aqueuse formée dans l'étape b) est retirée,
d) la phase organique formée dans l'étape b) est totalement ou partiellement réintroduite sous forme de reflux au niveau de la tête de la première colonne de distillation utilisée dans l'étape a) et une partie de la phase organique qui n'est pas utilisée comme reflux est retirée,
e) le gaz non condensé dans l'étape b) qui contient de l'acétate de vinyle est lavé dans une colonne de lavage avec au moins une solution aqueuse d'acide acétique à 90 % pour obtenir un acétate de vinyle contenant une solution acétique au fond,
f) une partie du produit de fond de la colonne de lavage utilisée dans l'étape e) est d'abord refroidie pendant le pompage par le dessus, puis réintroduite dans le fond de la colonne de lavage utilisée dans l'étape e), la partie restante du produit de fond est retirée et chauffée à au moins 30 °C, puis chargée dans la partie inférieure de la première colonne de distillation utilisée dans l'étape a),
g) un courant gazeux est retiré au niveau de la tête de la colonne de lavage de l'étape e), une partie étant évacuée sous forme de gaz d'échappement et la partie restante étant recyclée vers la zone de réaction,
h) le gaz d'échappement évacué dans l'étape g) passe à travers une solution aqueuse alcaline,
i) la solution aqueuse alcaline obtenue dans l'étape h) est régénérée par une augmentation de température à 100 à 120 °C,
j) le courant de CO₂ saturé avec de la vapeur d'eau résultant de l'étape i) est retiré,
k) le produit de fond de l'étape a) contenant de l'acétate de vinyle, de l'acétate d'éthyle, de l'acide acétique et de l'eau est chargé dans une cuve de récupération et le liquide pressurisé est dilaté de façon à former un gaz,
l) le liquide provenant de la dilatation de l'étape k) est chargé dans une deuxième colonne de distillation, et à partir d'une zone de concentration au-dessus de son fond un courant latéral contenant de l'acétate d'éthyle est retiré,
m) le produit de fond de l'étape 1) contenant de l'acide acétique et de l'eau est utilisé totalement ou partiellement pour le lavage du gaz de l'étape e),
n) la vapeur de tête de l'étape 1) est refroidie, de façon à former un condensat qui se sépare en une phase aqueuse et une phase organique,
o) la phase aqueuse formée dans l'étape n) est retirée,
p) une partie de la phase organique formée dans l'étape n) est recyclée sous forme de reflux au niveau de la tête de la deuxième colonne de distillation utilisée dans l'étape 1), et la partie restante est retirée,
q) la phase organique restante retirée dans l'étape d) est dilatée et le gaz formé pendant la dilatation est combiné au gaz formé dans l'étape k), et les courants gazeux combinés sont recyclés vers le procédé,
r) la phase organique résultant de l'étape q) est combinée à la phase organique résultant de l'étape n), et la partie retirée restante de la phase organique de l'étape p), qui n'est pas utilisée comme reflux, est introduite dans une troisième colonne de distillation,
s) le produit de tête de la troisième colonne de distillation de l'étape r) est refroidi et la fraction à bas point d'ébullition résultante et l'eau résultante sont séparées,
t) le produit de fond de la troisième colonne de distillation de l'étape r) est introduit dans une quatrième colonne de distillation,
u) l'acétate de vinyle pur est retiré de la tête de la quatrième colonne de distillation utilisée dans l'étape t), **caractérisé en ce que**
v) le produit de fond de la quatrième colonne de distillation est retiré, et une partie de celui-ci est chauffé dans un ou plusieurs échangeurs, qui fonctionnent avec le courant de CO₂ saturé avec de la vapeur d'eau obtenu dans l'étape j), et
w) le produit de fond ainsi chauffé est recyclé vers la partie inférieure de la quatrième colonne de distillation.

2. Procédé selon la revendication 1, **caractérisé en ce que** le courant de CO₂ saturé avec de la vapeur d'eau utilisé dans l'étape v) a une température de 90 à 120 °C.

3. Procédé selon la revendication 1 ou 2, **caractérisé en ce que** le courant de CO₂ saturé avec de la vapeur d'eau utilisé dans l'étape v) a une pression de 0,1 à 0,12 MPa.

4. Procédé selon une ou plusieurs des revendications 1 à 3, **caractérisé en ce que** la quatrième colonne de distillation est exploitée à une température de 70 à 100 °C.

5. Procédé selon une ou plusieurs des revendications 1 à 4, **caractérisé en ce que** le produit de fond de la quatrième colonne de distillation résultant de l'étape v) est retiré à une température de 70 à 100 °C.

6. Procédé selon la revendication 5, **caractérisé en ce qu'**une partie du produit de fond résultant de la quatrième colonne de distillation est chauffée à une température de 80 à 110 °C.
